# EUROPEAN PATENT SPECIFICATION

(11) **EP 4 112 010 B1**
(45) Date of publication and mention of the grant of the patent: **21.05.2025**
(21) Application number: 20920892.5
(22) Date of filing: 28.02.2020
(51) Int. Cl.: A61F 2/38

(54) **KNEE PROSTHESIS**
KNIEPROTHESE
PROTHÈSE DE GENOU

(43) Date of publication of application: 04.01.2023
(73) Proprietor: Beijing AK Medical Co., Ltd., 102200, Beijing (CN)
(72) Inventor: ZHANG, Weiping, Beijing 102200 (CN)
(74) Representative: Inchingalo, Simona
(86) International application number: PCT/CN2020/077251
(87) International publication number: WO 2021/168822

(56) References cited:
- CN-A- 101 214 175
- CN-A- 105 030 384
- CN-A- 106 109 063
- CN-A- 108 186 167
- CN-A- 109 620 480
- CN-A- 111 184 598
- CN-U- 205 849 594
- DE-A1- 19 529 824
- US-A1- 2001 021 877
- US-A1- 2003 153 977
- US-A1- 2004 138 755
- US-A1- 2008 009 950
- US-A9- 2018 185 134

## Description

### Technical Field

The present invention relates to a field of orthopaedic implants, and particularly relates to a knee prosthesis.

### Background

At present, an artificial knee prosthesis for total knee replacement includes a femoral condyle component, a spacer component and a tibial plateau component in most cases, and further includes a patellar component in some cases. A basic structure of the femoral condyle component includes medial and lateral condyle articular surfaces and a patellar slideway articular surface. The spacer component has two articular surfaces that correspond to the medial and lateral condyle articular surfaces of the femoral condyle component respectively so as to form a friction pair. A tibial plateau has a synostosis surface at a lower part, which is used to be located on a bone bed after tibial osteotomy, and the tibial plateau has a flat surface structure at an upper part, which is used to support a human body load transmitted by the spacer component. According to severity of knee damage of patients, a femoral condyle prosthesis is designed in various additional structural forms. For example, in the case of bicondylar femoral condyle prostheses of posterior cruciate-retaining total knee prostheses (commonly called CR-type), medial and lateral condyles are connected into a whole by a patellar slideway, and rear portions of both condyles are kept open to accommodate posterior cruciate ligaments. In the case of posterior stabilized total knee prostheses (commonly called PS-type) without retaining posterior cruciate ligaments, limiting beams are mounted at rear portions of both condyles, so as to match a central stand column of a tibial spacer, thereby enhancing stability of a prosthesis. In the cases of other semi-constraint and full-constraint designs, stability of an implanted knee prosthesis can be maintained by adding additional structures and components.

The various knee prostheses described above are basically consistent in mechanical design concepts of articular surfaces. That is, an osteotomy surface during proximal tibia osteotomy is perpendicular to a tibia anatomical axis, and an osteotomy surface during distal femur osteotomy forms an angle (acute lateral angle) of 84° with a femur anatomical axis. A lower limb force line vertically passes the middle of a joint line of a knee prosthesis formed after an operation. In other words, the joint line is perpendicular to a mechanical axis and is slightly inclined relative to a gravity line. The operation has become a classic operation for knee replacement over the years. The inventor has found that movement and stress states of a knee prosthesis in the prior art are quite different from those of a physiological knee of a human body and will lead to increase in wear of the knee prosthesis, which will be described in detail below.

A joint line of the physiological knee of a human body, a line connecting distal ends of medial and lateral condyles of a knee or a line connecting high points of medial and lateral plateaus of a tibia, is perpendicular to the gravity line, a force line pointing to the earth's core, or parallel to a horizontal plane. However, in the above-mentioned designs of various knee prostheses, the joint line forms an inclination angle of about 3° with the horizontal plane. In this way, a surface of a femoral condyle prosthesis making contact with a spacer of a tibial plateau produces a transverse shear force under the action of gravity, and the shear force increases additional wear risks during movement of a joint pair.

During knee flexion and extension, a tibia can rotate relative to a femur. Normally, the rotary movement of the physiological knee of a human body refers to rotation around an instantaneous center line perpendicular to the ground in the horizontal plane. However, after a knee prosthesis operation, the rotary movement is changed to rotation around an instantaneous center line parallel to the tibia anatomical axis in an inclined plane forming an angle of 3° with the horizontal plane. In this case, tension stress of all medial and lateral ligaments involved in keeping knees stable is obviously different from a normal physiological condition before an operation. This will lead to abnormal gait and proprioception of a patient during flexion and extension, and will produce obvious sense of disharmony and increase wear of medial or lateral prosthesis articular surfaces heavily stressed.

When a knee is highly flexed, a spacer of a knee prosthesis in the prior art cannot adjust an angle of rolling forward and backward, leading to edge contact between a rear edge of a posterior condyle of a femoral condyle prosthesis and a spacer and even bite of the spacer by the rear edge of the posterior condyle of the femoral condyle prosthesis, and further shortening the service life of the knee prosthesis.

In knee joint movement, a femoral condyle component slides and rotates back and forth as well as left and right relative to a tibial plateau. In the prior art, a spacer of the tibial plateau of the knee prosthesis can also move slightly, but it cannot follow a movement track of a femoral condyle in all directions. Especially, during rotation, a concave articular surface of the spacer which originally fitted a femoral prosthesis condyle surface inevitably presses or collides with a femoral prosthesis condyle side surface, thus accelerating wear.

### Summary

A main objective of the present invention is to provide a knee prosthesis, so as to solve the a femoral prosthesis condyle surface inevitably presses or collides with a femoral prosthesis condyle side surface, thus accelerating wear.

Examples of a knee prosthesis according to the prior art are known from US 2004/138755A1, US2018/185134A9, US2008/009950A1, and DE195 28 824A1.

### Summary

A main objective of the present invention is to provide a knee prosthesis, so as to solve the problem that a knee prosthesis is prone to wear in related technologies.

The invention is as defined in claim 1.

To achieve the objective, the present invention provides a knee prosthesis. The knee prosthesis includes: a tibial plateau; a femoral condyle component, provided above the tibial plateau, wherein the femoral condyle component includes a medial condyle articular surface and a lateral condyle articular surface, and there is a height difference H between the medial condyle articular surface and the lateral condyle articular surface; and a spacer component between the tibial plateau and the femoral condyle component.

Furthermore, the spacer component includes a medial spacer cooperating with the medial condyle articular surface and a lateral spacer cooperating with the lateral condyle articular surface. An upper surface of the tibial plateau is provided with a first spherical convex surface at a position corresponding to the lateral spacer. The lateral spacer is movably arranged on the tibial plateau. A lower surface of the lateral spacer is provided with a first spherical concave surface cooperating with the first spherical convex surface.

In some embodiments, the height difference H is between 0.5 mm and 5 mm.

In some embodiments, the lateral condyle articular surface is provided with a second spherical convex surface. The spacer component is provided with a second spherical concave surface cooperating with the second spherical convex surface.

In some embodiments, the lateral condyle articular surface is further provided with a first diameter-variable convex curved surface.

In some embodiments, the medial condyle articular surface is provided with a second diameter-variable convex curved surface. The spacer component is provided with a first diameter-variable concave curved surface cooperating with the second diameter-variable convex curved surface.

In some embodiments, the upper surface of the tibial plateau is provided with a first flat surface at a position corresponding to the medial condyle articular surface, and a lower surface of the medial spacer is provided with a second flat surface cooperating with the first flat surface. Alternatively, the upper surface of the tibial plateau is provided with a third spherical convex surface at a position corresponding to the medial condyle articular surface, and a lower surface of the medial spacer is surrounded by the first medial wall and the peripheral side wall. The medial spacer is arranged inside a second space surrounded by the second medial wall and the peripheral side wall. The first medial wall protrudes toward the lateral spacer. The second medial wall protrudes toward the medial spacer.

According to the invention, the tibial plateau includes a plateau body, a peripheral side wall arranged on a circumferential edge of the plateau body, as well as a first medial wall and a second medial wall that are arranged inside the peripheral side wall. The lateral spacer is movably arranged inside a first space surrounded by the first medial wall and the peripheral side wall. The medial spacer is arranged inside a second space surrounded by the second medial wall and the peripheral side wall.

In some embodiments, the medial spacer is movably arranged inside the second space.

In some embodiments, a size of the first space is larger than a size of the lateral spacer, so as to make the lateral spacer slide and/or rotate relative to the tibial plateau. A sieze of the second space is larger than a size of the medial spacer, so as to make the medial spacer slide and/or rotate relative to the tibial plateau.

In some embodiments, the medial spacer is fixedly arranged on the tibial plateau. The spacer component further includes a stand column arranged on the medial spacer.

In some embodiments, the femoral condyle component further includes a patellar slideway articular surface connected between the medial condyle articular surface and the lateral condyle articular surface, as well as a limiting beam between a posterior condyle of the medial condyle articular surface and a posterior condyle of the lateral condyle articular surface. The stand column is capable of limiting the limiting beam.

In some embodiments, the tibial plateau is provided with a shaft. The stand column is internally provided with an inner hole. The shaft is inserted into the inner hole.

In some embodiments, the tibial plateau further includes an intramedullary rod arranged below the plateau body. There is an included angle α between a vertical line perpendicular to the upper surface of the tibial plateau and the intramedullary rod.

According to use of the technical solution of the present invention, the tibial plateau is arranged on a tibia after osteotomy, the femoral condyle component is arranged on a femur after osteotomy, and the spacer component is arranged between the tibial plateau and the femoral condyle component. The femoral condyle component includes the medial condyle articular surface and the lateral condyle articular surface. The medial condyle articular surface and the lateral condyle articular surface both contact with the spacer component. When a patient is in an upright position and a flexed position, a height difference between a farthest point of the medial condyle articular surface and a farthest point of the lateral condyle articular surface is H, and the farthest point of the medial condyle articular surface is slightly lower than that of the lateral condyle articular surface when viewed in a coronal position. The height difference H is set to make a line connecting farthest points of medial and lateral condyles of a distal end of a femur, which is a joint line, parallel to a horizontal plane and no longer perpendicular to a tibia anatomical axis. The arrangement mode may effectively avoid transverse shear stress generated by contact surfaces between the femoral condyle component and the spacer component, and between the spacer component and the tibial plateau under the action of gravity. Therefore, the technical solution of the disclosure effectively solves the problem that the knee prosthesis is prone to wear caused by the shear stress in related technologies. In addition, after the technical solution of the disclosure is used, rotary movement between a tibia and a femur during knee flexion and extension of the patient refers to rotation around an instantaneous center line perpendicular to the ground in an approximate horizontal plane, such that the patient has normal gait and movement feeling. When a knee is highly flexed, the spacer component may slide backward along a spherical convex surface of the upper surface of the tibial plateau, so as to prevent a rear edge of a femoral condyle prosthesis from making side contact with a spacer and biting the spacer. In addition, during knee movement, the spacer component may follow the femoral condyle component to slide and rotate in all directions along a spherical convex/flat surface of the upper surface of the tibial plateau, such that contact surfaces of the spacer component and the femoral condyle component fit each other, and lateral pressing or collision may be avoided.

### Brief Description of the Drawings

The drawings of the description, which form a part of the disclosure, are used to provide further understanding of the present invention, and illustrative embodiments of the present invention and the description thereof are used to explain the present invention, which are not intended to unduly limit the present invention. In the drawings:
Fig. 1 shows a structural schematic diagram of an exploded view of a first embodiment of a knee prosthesis according to the present invention;
Fig. 2 shows a structural schematic diagram of an exploded view of another view angle of the knee prosthesis in Fig. 1;
Fig. 3 shows a cutaway view of a local structure of the knee prosthesis in Fig. 2;
Figs. 4a and 4b show schematic diagrams of movement of a spacer component of the knee prosthesis in Fig. 1;
Fig. 5 shows a schematic diagram of a front view of a femoral condyle component of the knee prosthesis in Fig. 1;
Fig. 6 shows a schematic diagram of a cutaway view of an A-A direction of the femoral condyle component in Fig. 5;
Fig. 7 shows a schematic diagram of a cutaway view of a B-B direction of the femoral condyle component in Fig. 5;
Fig. 8 shows a schematic diagram of a curve of a medial condyle articular surface in Fig. 6;
Fig. 9 shows a schematic diagram of a curve of a lateral condyle articular surface in Fig. 7;
Fig. 10 shows a schematic diagram of a side view of a tibial plateau of the knee prosthesis in Fig. 1;
Fig. 11 shows cutaway views of the knee prosthesis in Fig. 1 and a knee prosthesis in the prior art;
Fig. 12 shows cutaway views of the knee prosthesis in Fig. 1 and the knee prosthesis in the prior art in another state;
Fig. 13 shows structural schematic diagrams of exploded views of the knee prosthesis in Fig. 1 and the knee prosthesis in the prior art;
Fig. 14 shows structural schematic diagrams of exploded views of the knee prosthesis in Fig. 11 and the knee prosthesis in the prior art from another view angle;
Fig. 15 shows schematic diagrams of movement states of the knee prosthesis in Fig. 11 and the knee prosthesis in the prior art;
Fig. 16 shows a structural schematic diagram of an exploded view of an example of a knee prosthesis that does not form part of the present invention;
Fig. 17 shows a cutaway view of a local structure of the knee prosthesis in Fig. 16;
Fig. 18 shows a structural schematic diagram of an exploded view of a second embodiment of the knee prosthesis according to the present invention; and
Fig. 19 shows a cutaway view of a local structure of the knee prosthesis in Fig. 18.

The above figures include the following reference numerals:
10: tibial plateau; 11: first spherical convex surface; 12: first flat surface; 13: third spherical convex surface; 14: plateau body; 15: peripheral side wall; 161: first medial wall; 162: second medial wall; 17: shaft; 18: intramedullary rod; 20: femoral condyle component; 21: medial condyle articular surface; 211: second diameter-variable convex curved surface; 22: lateral condyle articular surface; 221: second spherical convex surface; 222: first diameter-variable convex curved surface; 23: patellar slideway articular surface; 24: limiting beam; 30: spacer component; 31: first spherical concave surface; 32: second flat surface; 33: third spherical concave surface; 34: medial spacer; 35: lateral spacer; 36: stand column; 361: inner hole; 37: first diameter-variable concave curved surface; and 38: second spherical concave surface.

### Detailed Description of the Embodiments

The technical solutions of the embodiments of the present invention will be clearly and completely described below with reference to the accompanying drawings of the embodiments of the present invention. Apparently, the described embodiments are merely a part rather than all of the embodiments of the present invention. The following description of at least one exemplary embodiment is merely illustrative in nature and in no way serves as any limitation of the present invention and its application or uses. Based on the embodiments of the present invention, other various embodiments obtained by those of ordinary skill in the art without making creative efforts fall within the scope of protection of the present invention.

It should be noted that the terms used herein only serve to describe specific implementations and are not intended to limit the illustrative implementations according to the disclosure. As used herein, the singular is intended to include the plural unless the context clearly dictates, and furthermore, it should be understood that the terms "include" and/or "comprise", when used in the description, specify the presence of features, steps, operations, devices, assemblies, and/or combinations thereof.

It should be noted that the relative arrangement, numerical expressions and numerical values of components and steps described in the embodiments do not limit the scope of the present invention unless otherwise specified. Moreover, it should be understood that for convenience of description, sizes of each part shown in the drawings are not drawn according to a real proportional relation. Technologies, methods and devices known to those of ordinary skill in related fields may not be discussed in detail, but in appropriate cases, they should be regarded as part of the description. In all the examples shown and discussed herein, any specific value should be interpreted as exemplary only, not as a limitation. Therefore, other examples of exemplary embodiments may have different values. It should be noted that similar numerals and letters denote similar items in the following accompanying drawings, and therefore, once an item is defined in one accompanying drawing, it needs not be further discussed in the subsequent accompanying drawings.

After experiments, the inventor finds specific causes of wear in the prior art. In the prior art, there is an included angle of 3° between a joint line of a knee prosthesis and a horizontal plane, contact surfaces between a femoral condyle component and a spacer component, and between the spacer component and a tibial plateau may generate a transverse shear force under the action of gravity, and the shear force increases additional wear risks during movement of the knee prosthesis.

In addition, during knee flexion and extension, a tibia rotates relative to a femur. Under a normal physiological condition, the rotary movement refers to rotation around an instantaneous center line perpendicular to the ground in a horizontal plane. However, after an operation, the rotary movement is changed to rotation around an instantaneous center line parallel to a tibia anatomical axis in an inclined plane having an angle of 3° with the horizontal plane. In this case, tension stress of all medial and lateral ligaments involved in keeping a knee stable is obviously different from a normal physiological condition before an operation. In this way, abnormal gait and proprioception of a patient during flexion and extension may be caused, an obvious sense of disharmony may be generated, and wear of medial or lateral prosthesis articular surfaces heavily stressed may be increased.

When the knee is highly flexed, a spacer of a knee prosthesis in the prior art cannot adjust a pitch angle before and after, leading to edge contact between a rear edge of a posterior condyle of a femoral condyle prosthesis and a spacer, and even bite of the spacer, which may shorten a service life of a joint prosthesis.

In knee joint movement, a femoral condyle may slide and rotate back and forth, left and right relative to the tibial plateau. In the prior art, a spacer of the knee prosthesis may also move slightly, but cannot follow a movement track of the femoral condyle in all directions. Especially, during rotation, the spacer originally fits a concave articular surface of a femoral prosthesis condyle surface. However, along with rotation, a femoral prosthesis condyle side surface may press or collide with the spacer, thus accelerating the wear.

The inventor conducts relevant analysis on the femoral condyle, a knee and a tibia of a human body. At present, the medical profession agrees that there are three mechanical alignment lines of human lower limbs, that is, a gravity line, a mechanical axis and an anatomical axis. In addition, there is another important marker line-joint line (that is, a line connecting distal ends of medial and lateral condyles of a knee or a line connecting high points of medial and lateral plateaus of a tibia lower limb force line bone). The gravity line is a force line that passes the center of gravity of the human body and then vertically points to the earth's core (which is parallel to a plumb line formed by the gravity). The mechanical axis is a force line passing a center of a femur, a center of a knee and then a center of an ankle. The anatomical axis may be understood to approximate a midline of diaphyses of a femur and a tibia from a coronal position. The "approximate" is used because an axis of the diaphysis of the femur is not a straight line when viewed in a three-dimensional space. The joint line is usually in a nearly horizontal position, that is, perpendicular to the plumb line. Under a normal physiological condition, when the human body stands, a line connecting the center of the femur, the center of the knee and the center of the ankle should be in a same straight line. The straight line is a mechanical axis or a force line of a lower limb. The mechanical axis has an average outward inclination angle of 3° with the gravity line. A femur anatomical axis passing the diaphysis of the femur has an average outward inclination angle of about 6° with the mechanical axis at the center of the knee. The tibia anatomical axis has an average outward inclination angle of 3° with the gravity line (under the normal physiological condition, the tibia anatomical axis coincides with the mechanical axis of the lower limb). A tibial angle is a lateral angle formed by the femur anatomical axis and the tibia anatomical axis at the center of the knee, which is 174°. An average angle between the femur anatomical axis and the gravity line is 9°.

Based on what is described above, in the disclosure, there is a height difference H between a medial condyle articular surface and a lateral condyle articular surface, that is, a farthest point of the medial condyle articular surface is slightly lower than a farthest point of the lateral condyle articular surface when viewed in a coronal position. In this way, when a knee prosthesis is implanted, an osteotomy surface on a tibial side is made parallel to the horizontal plane. Then, a joint line of a knee prosthesis is made parallel to the horizontal plane after an operation, thereby not influencing a physiological included angle between the femur anatomical axis and the mechanical axis.

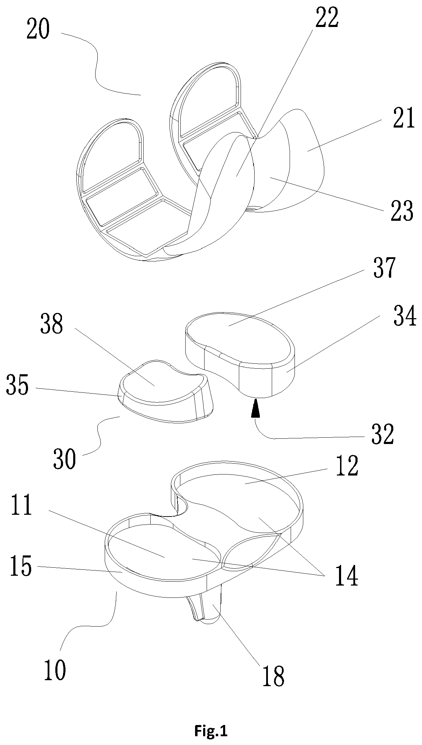

As shown in Figs. 1-5, in a first embodiment of the invention, a knee prosthesis includes: a tibial plateau 10, a femoral condyle component 20 and a spacer component 30. The femoral condyle component 20 is located above the tibial plateau 10. The femoral condyle component 20 includes a medial condyle articular surface 21 and a lateral condyle articular surface 22. There is a height difference H between the medial condyle articular surface 21 and the lateral condyle articular surface 22. The spacer component 30 is located between the tibial plateau 10 and the femoral condyle component 20.

With the technical solution of the present invention used, the tibial plateau 10 is arranged on a tibia after osteotomy. The femoral condyle component 20 is arranged on a femur after osteotomy. The spacer component 30 is arranged between the tibial plateau 10 and the femoral condyle component 20. The femoral condyle component 20 includes the medial condyle articular surface 21 and the lateral condyle articular surface 22. The medial condyle articular surface 21 and the lateral condyle articular surface 22 both contact with the spacer component 30. When a patient is in an upright position and a flexed position, a height difference between a farthest point of the medial condyle articular surface 21 and a farthest point of the lateral condyle articular surface 22 is H, that is, the farthest point of the medial condyle articular surface 21 is slightly lower than the farthest point of the lateral condyle articular surface 22, and the farthest point of the medial condyle articular surface 21 is slightly lower than the farthest point of the lateral condyle articular surface 22. The height difference H is set such that a joint line formed by connecting the farthest point of the medial condyle articular surface 21 and the farthest point of the lateral condyle articular surface 22 is not perpendicular to a tibia anatomical axis during use, but may be parallel to a horizontal plane. The arrangement mode may effectively avoid transverse shear stress generated by contact surfaces between the femoral condyle component 20 and the spacer component 30, and between the spacer component 30 and the tibial plateau 10 under the action of gravity. Therefore, the technical solution of the disclosure effectively solves the problem that a knee prosthesis is prone to wear caused by the shear stress in related technologies. In addition, after the technical solution of the disclosure is used, rotary movement between a tibia and a femur during knee flexion and extension of the patient refers to rotation around an instantaneous center line perpendicular to the ground in an approximate horizontal plane, such that the patient has normal gait and movement feeling.

When a knee is viewed in a coronal position of a human body to be in an upright position and a flexed position, there is a height difference H between the farthest point of the medial condyle articular surface and the farthest point of the lateral condyle articular surface of the femoral condyle component, that is, the farthest point of the medial condyle articular surface is 0.5 mm-5 mm lower than the farthest point of the lateral condyle articular surface, so as to conform to an anatomical shape of a femoral condyle of a human body. The feature extends from a farthest point of a condyle articular surface to a posterior condyle. A specific height difference should be selected according to specific data of different patients.

An inner surface of the femoral condyle component 20 making contact with an osteotomy surface on a femoral side is a femur and condyle integration surface. The femur and condyle integration surface is attached to the osteotomy surface on a femoral side during an operation. Through bonding of bone cement or connection between a rough surface of a bone integration surface and a porous structure surface, the knee prosthesis may be integrated with a physiological bone of the patient in a future rehabilitation process, so as to stabilize the prosthesis for a long term.

The femoral condyle component 20, the spacer component 30 and the tibial plateau 10 are usually made of medical metals (such as cobalt alloy, titanium and titanium alloy, tantalum and tantalum alloy and magnesium alloy), medical ceramic materials (such as alumina ceramic, zirconia ceramic, silicon carbide ceramic and silicon nitride ceramic), and high polymer materials.

The inventor finds that the knee may be regarded as a tibiofemoral joint composed of medial and lateral condyles of a distal end of a femur, medial and lateral plateaus as well as medial and lateral meniscuses of a proximal end of a tibia, from an anatomical structure. A lateral articular surface of the tibial plateau is bowl-shaped, and a convex lateral condyle articular surface of a femur and a concave lateral articular surface of the tibial plateau fit each other, such that a lateral joint pair of the knee is capable of flexing and extending flexibly on a sagittal plane. A recess on a medial articular surface of the tibial plateau is somewhat like a broad basin surrounded by meniscuses. The first 1/3 of the medial articular surface of the tibial plateau is a concave surface that rises gradually, while the second 2/3 is a concave surface that falls gradually. When cooperating with a medial femoral condyle, the medial articular surface of the tibial plateau cannot constantly and completely fit the medial femoral condyle during flexion and extension activities, but allows the medial femoral condyle to slide back and forth to a certain extent and roll with a plurality of instantaneous activity centers.

Based on the above research, the embodiment makes the following improvements to the prior art:
As shown in Figs. 1-9, in the first embodiment, the lateral condyle articular surface 22 is provided with a second spherical convex surface 221, and a lateral spacer 35 is provided with a second spherical concave surface 38 cooperating with the second spherical convex surface 221. The second spherical convex surface 221 contacts with the second spherical concave surface 38. The second spherical convex surface 221 is capable of sliding and rotating on the second spherical concave surface 38. The second spherical convex surface 221 fits the second spherical concave surface 38 well, such that sliding and rotation of the second spherical convex surface 221 may be more stable.

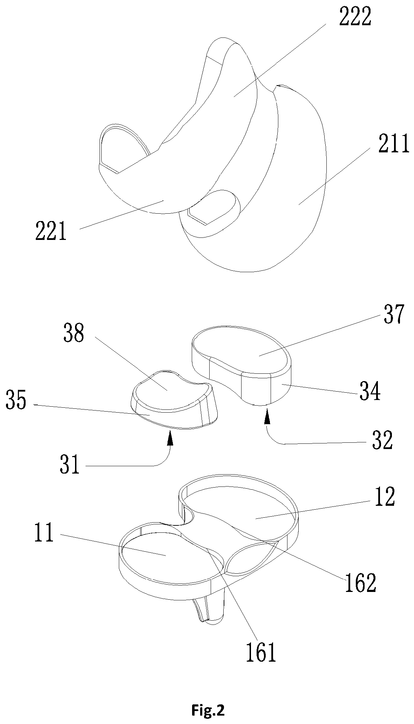

As shown in Figs. 2, 3 and 9, in the first embodiment, the lateral condyle articular surface 22 is further provided with a first diameter-variable convex curved surface 222. The first diameter-variable convex curved surface 222 is connected to the second spherical convex surface 221, such that the lateral condyle articular surface 22 may slide in a larger space. In addition, the first diameter-variable convex curved surface 222 is capable of restraining lateral sides of a patella and a patellar ligament of the patient.

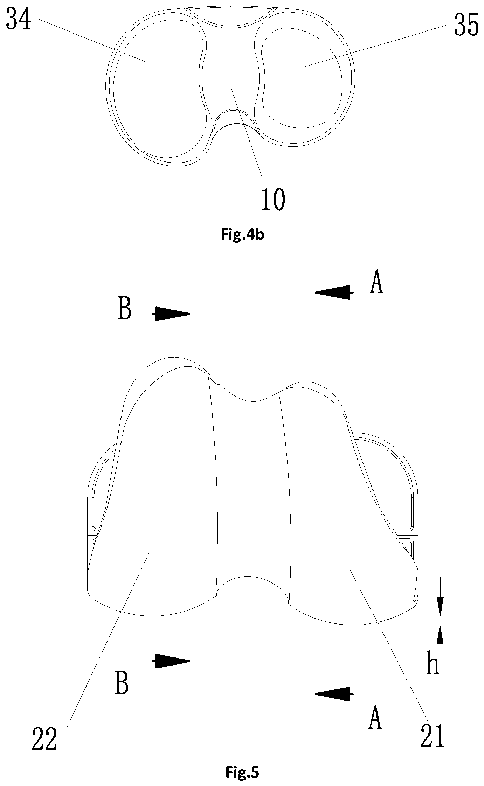

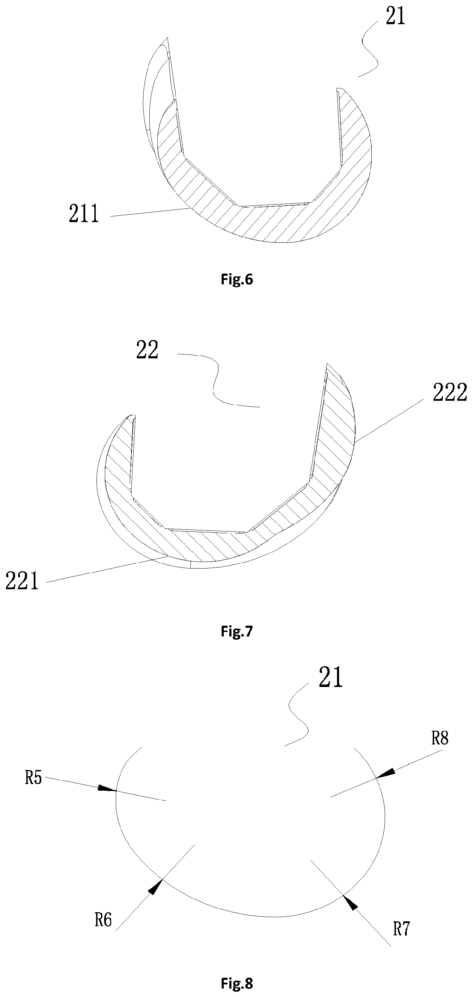

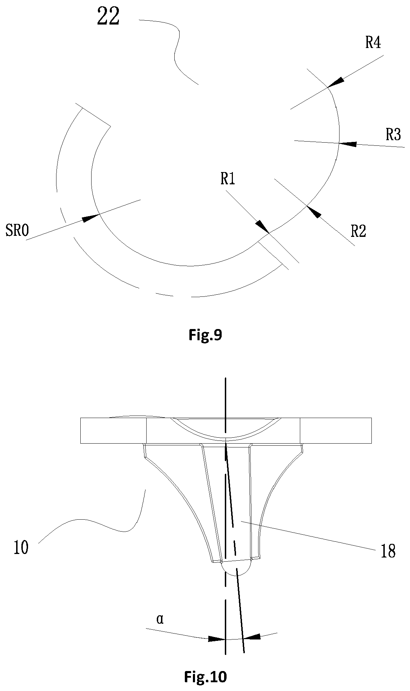

Figs. 6 and 7 are cutaway views of A-A and B-B directions in Fig. 5 respectively. Fig. 6 mainly shows an outline of the medial condyle articular surface 21. Fig. 7 mainly shows an outline of the lateral condyle articular surface 22. Fig. 8 shows a borderline of the medial condyle articular surface 21. Fig. 9 shows a borderline of the lateral condyle articular surface 22. As shown in Fig. 9, regarding the borderline of the lateral condyle articular surface 22, one part corresponds to the second spherical convex surface 221, and the other part corresponds to the first diameter-variable convex curved surface 222. A radius of an arc corresponding to the second spherical convex surface 221 is SR0. A borderline corresponding to the first diameter-variable convex curved surface 222 includes four arcs. Radii of four arcs are R1, R2, R3 and R4 sequentially. R1 indicates a position of an inward concave arc, and the other arcs corresponding to R2, R3 and R4 are outward convex arcs. In Fig. 9, a marked size relation of SR0-R4 may be adjusted according to an actual condition. As shown in Fig. 8, a borderline corresponding to a second diameter-variable convex curved surface 211 also includes four arcs. Radii of four arcs are R5, R6, R7 and R8 sequentially. The arcs are all outward convex arcs. In Fig. 8, a marked size relation of R5-R9 may be adjusted according to an actual condition. Certainly, as a feasible implementation, regarding the borderline of the lateral condyle articular surface 22, the borderline corresponding to the first diameter-variable convex curved surface 222 may include two, three, five or more arcs, or curves in other forms, as long as smooth and stable fit between the first diameter-variable convex curved surface 222 and the lateral spacer 35 may be satisfied. Similarly, regarding the borderline of the medial condyle articular surface 21, the borderline corresponding to the second diameter-variable convex curved surface 211 may include two, three, five or more arcs, or curves in other forms, as long as smooth and stable fit between the second diameter-variable convex curved surface 211 and the medial spacer 34 may be satisfied.

As shown in Figs. 1-3, in the first embodiment, the medial condyle articular surface 21 is provided with the second diameter-variable convex curved surface 211, and the medial spacer 34 is provided with a first diameter-variable concave curved surface 37 cooperating with the second diameter-variable convex curved surface 211. When the knee is in the upright position, the second diameter-variable convex curved surface 211 fits the first diameter-variable concave curved surface 37, so as to stabilize the knee. When the knee starts to flex, the second diameter-variable convex curved surface 211 makes local surface contact or even line contact with the first diameter-variable concave curved surface 37, so as to allow a certain sliding or rotational displacement between the femoral condyle component 20 and the spacer component 30. An anterior upper half of the medial condyle articular surface 21 is a convex diameter-variable curved surface and has a smooth transitive surface with an adjacent patellar slideway articular surface 23, so as to restrain medial sides of a patella and a ligament thereof. The medial condyle articular surface 21 of the femoral condyle component 20 is a convex continuous diameter-variable curved surface from an anterior lower half to a posterior condyle. The convex continuous diameter-variable curved surface makes contact with and matches the first diameter-variable concave curved surface 37, which is downward concave, of an upper surface of the medial spacer 34 of the spacer component 30. The second diameter-variable convex curved surface 211 fits the first diameter-variable concave curved surface 37, such that the medial condyle articular surface 21 may slide and rotate more stably, and a better use effect is provided for the patient.

As shown in Figs. 1-3, in the first embodiment, the spacer component 30 includes the medial spacer 34 cooperating with the medial condyle articular surface 21 and the lateral spacer 35 cooperating with the lateral condyle articular surface 22. An upper surface of the tibial plateau 10 is provided with a first spherical convex surface 11 at a position corresponding to the lateral spacer 35. The lateral spacer 35 is movably arranged on the tibial plateau 10. A lower surface of the lateral spacer 35 is provided with a first spherical concave surface 31 cooperating with the first spherical convex surface 11. The first spherical convex surface 11 is capable of fitting the first spherical concave surface 31. The first spherical concave surface 31 is capable of sliding and rotating on the first spherical convex surface 11. When the knee prosthesis is in the flexed position, the lateral condyle articular surface 22 is capable of always keeping a large contact area with the lateral spacer 35, such that flexion stability is better, and moreover, service lives of the lateral condyle articular surface 22 and the spacer component 30 may be prolonged.

As shown in Figs. 1-3, in the first embodiment, the upper surface of the tibial plateau 10 is provided with a first flat surface 12 at a position corresponding to the medial condyle articular surface 21. A lower surface of the medial spacer 34 is provided with a second flat surface 32 cooperating with the first flat surface 12. The first flat surface 12 matches the second flat surface 32 in an attached manner. The second flat surface 32 is capable of moving or rotating relative to the first flat surface 12, thereby allowing movement of the medial condyle articular surface 21 relative to the spacer component 30. In this way, the femoral condyle component 20 has a movement effect closer to a physiological knee, which brings a better use experience to the patient.

As shown in Figs. 1-3, in the first embodiment, the spacer component 30 includes the medial spacer 34 cooperating with the medial condyle articular surface 21 and the lateral spacer 35 cooperating with the lateral condyle articular surface 22. The medial spacer 34 is separated from the lateral spacer 35. Both the medial spacer 34 and the lateral spacer 35 have their own movement modes. The separated arrangement may effectively avoid mutual restriction and interference between the medial spacer 34 and the lateral spacer 35, so as to make the knee prosthesis have a movement effect that better conforms to the physiological knee of the human body. When the knee prosthesis flexes to reach a maximum angle, the lateral spacer 35 moves to a rear edge of the tibial plateau 10. In this case, an upward concave spherical surface of the lower surface of the lateral spacer 35 still fits an upward convex spherical surface of the upper surface of a lateral side of the tibial plateau well, and the lateral spacer 35 has a backward inclination angle. The inclination angle prevents the rear edge of the femoral condyle component 20 from making contact with and biting the spacer component 30. Certainly, as a feasible implementation, the medial spacer 34 and the lateral spacer 35 may be connected together or integrated into a structure. In this case, it should be noted that considering a movement difference between the medial spacer 34 and the lateral spacer 35, the two need to be flexibly connected, so as to avoid mutual influences. In the embodiment, during knee movement, the tibial plateau 10 and the spacer component 30 follow the femoral condyle component 20, to slide and rotate in all directions, and surfaces of the spacer component 30 and the femoral condyle component 20 fit each other, so as to avoid pressing or collision. Meanwhile, when the knee is highly flexed, a rear edge of a posterior condyle no longer makes edge contact with the spacer component, so as to avoid bite.

In an embodiment not shown in the figure, the medial spacer may be integrated with the lateral spacer, and a structure of a spacer may be selected according to conditions of the patient.

As shown in Figs. 1-4b, in the first embodiment, the tibial plateau 10 includes a plateau body 14, a peripheral side wall 15 arranged on a circumferential edge of the plateau body 14, as well as a first medial wall 161 and a second medial wall 162 that are arranged inside the peripheral side wall 15. The medial spacer 34 is arranged inside a second space surrounded by the second medial wall 162 and the peripheral side wall 15. The lateral spacer 35 is arranged inside a first space surrounded by the first medial wall 161 and the peripheral side wall 15. The second medial wall 162 protrudes toward the medial spacer 34. The first medial wall 161 protrudes toward the lateral spacer 35. The peripheral side wall 15 cooperates with the first medial wall 161 and the second medial wall 162, so as to form the first space and the second space. The peripheral side wall 15, the first medial wall 161 and the second medial wall 162 have simple structures and are easy to form. The first medial wall 161 and the second medial wall 162 are in arc shapes that protrude outwards. Therefore, the first medial wall 161 and the second medial wall 162 may provide moving spaces for the lateral spacer 35 and the medial spacer 34 which rotate around an instantaneous center along with the femoral condyle component 20, so as to avoid collision between the medial walls and the spacers. The peripheral side wall 15 cooperates with the first medial wall 161 and the second medial wall 162, so as to prevent dislocation of the spacer component 30.

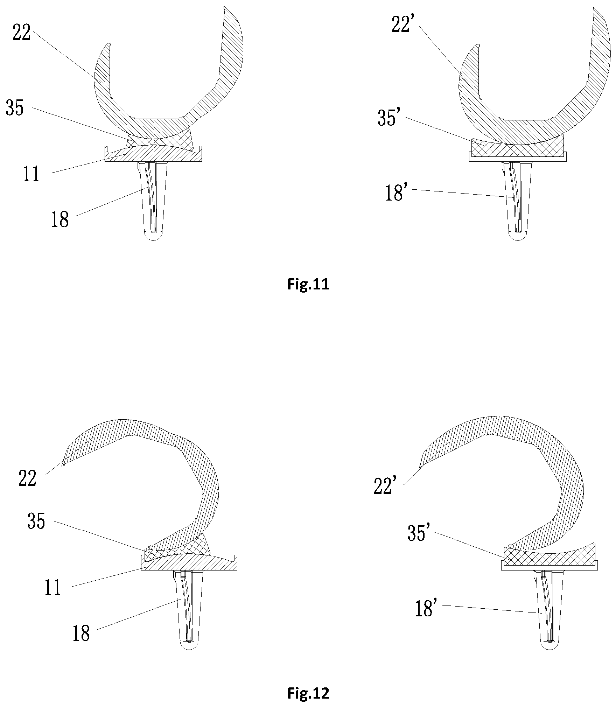

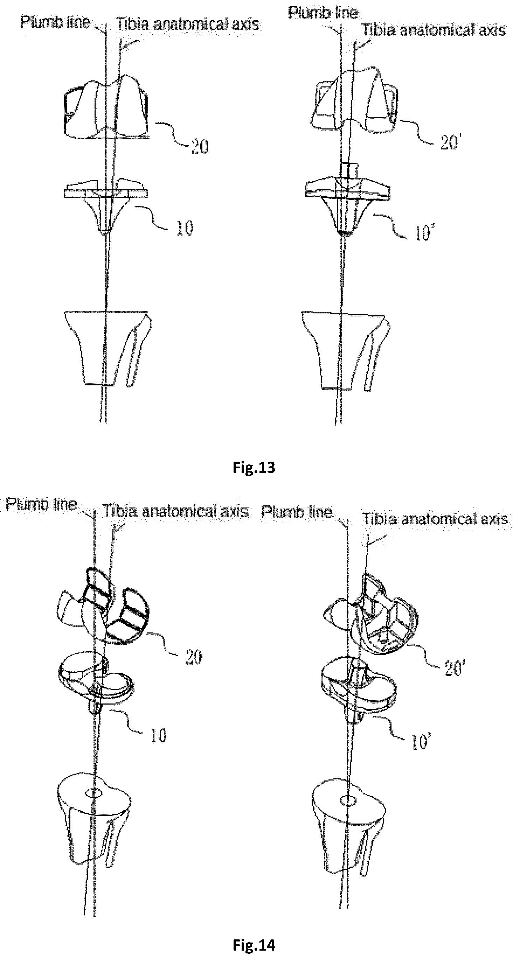

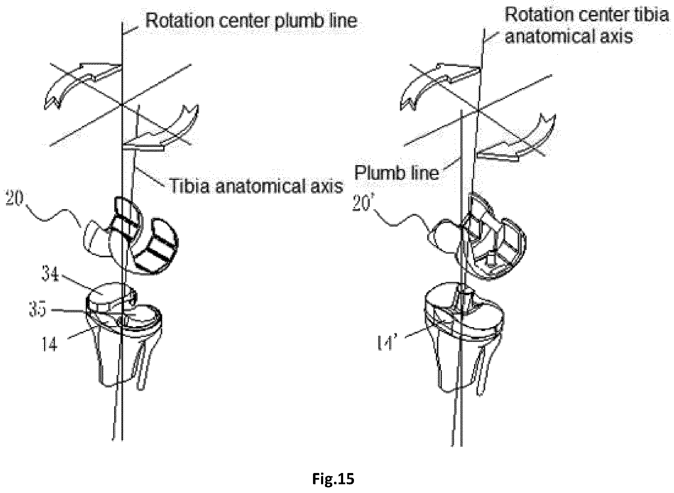

As shown in Figs. 1, 4a, 4b, and 11-15, in the first embodiment, the tibial plateau 10 includes the plateau body 14, the peripheral side wall 15 arranged on the circumferential edge of the plateau body 14, as well as the first medial wall 161 and the second medial wall 162 that are arranged inside the peripheral side wall 15. The medial spacer 34 is movably arranged inside the second space surrounded by the second medial wall 162 and the peripheral side wall 15. The lateral spacer 35 is movably arranged inside the first space surrounded by the first medial wall 161 and the peripheral side wall 15. The first space and the second space limit the lateral spacer 35 and the medial spacer 34 respectively, so as to prevent the medial spacer 34 and the lateral spacer 35 from moving excessively, resulting in damage of the knee prosthesis.

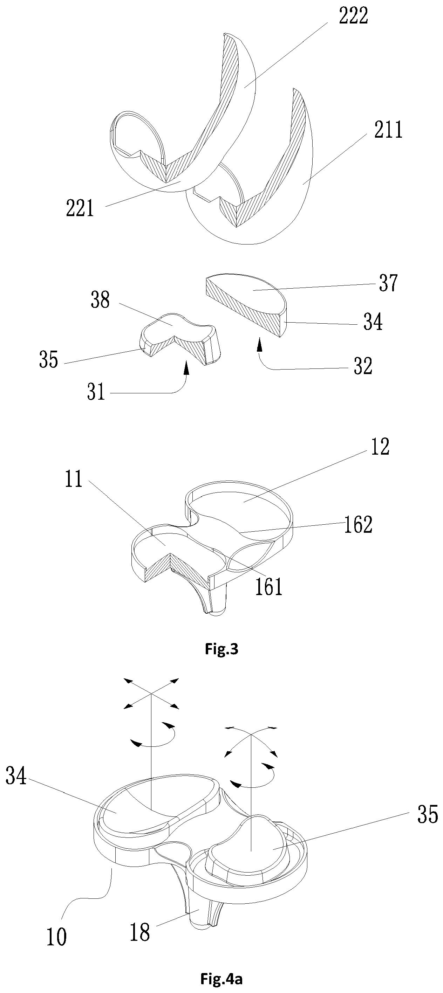

As shown in Figs. 4a and 4b, in the first embodiment, the first space has a larger size than the lateral spacer 35, so as to make the lateral spacer 35 slide and/or rotate relative to the tibial plateau 10. The second space has a larger size than the medial spacer 34, so as to make the medial spacer 34 slide and/or rotate relative to the tibial plateau 10. The second space and the first space have larger sizes than the medial spacer 34 and the lateral spacer 35 respectively, such that the medial spacer 34 and the lateral spacer 35 are capable of moving in the second space and the first space respectively. Further, the spacer component 30 has a larger moving space, thus effectively improving performance of the knee prosthesis.

As shown in Figs. 1, 10, 12 and 13, in the first embodiment, the tibial plateau 10 further includes an intramedullary rod 18 arranged below the plateau body 14, and there is an included angle α between a vertical line perpendicular to the upper surface of the tibial plateau 10 and the intramedullary rod 18. An average included angle between a femur anatomical axis and a gravity line is 9°. An average included angle between a tibia anatomical axis and the gravity line is 3°. However, under a normal physiological condition, the joint line of the knee (that is, a line connecting distal ends of medial and lateral condyles or a line connecting high points of medial and lateral plateaus of a tibia) is nearly a horizontal line. The reason for formation of the horizontal line is that a femur has a medial condyle longer than a lateral condyle in anatomy, such that an included angle (a lateral acute angle) between the line connecting the distal ends of the medial and lateral condyles and the femur anatomical axis in a coronal position is about 81°. In addition, an included angle (a medial acute angle) between the line connecting the high points of the medial and lateral plateaus of the tibia and the tibia anatomical axis is about 87°. When there is a certain relative rotation between a femoral condyle and the tibial plateau during flexion and extension, a rotation surface is in the horizontal plane, and an instantaneous rotation center line is a plumb line. Therefore, the included angle α is set between 1° and 5°, preferably 3°. The included angle α is set to make the plateau body 14 parallel to the horizontal plane after the operation of the patient, thereby effectively avoiding shear stress between the spacer component 30 and the tibial plateau 10 and shear stress between the tibial plateau 10 and the tibia. Therefore, the knee prosthesis has a better use effect and effectively improves a use experience of the patient.

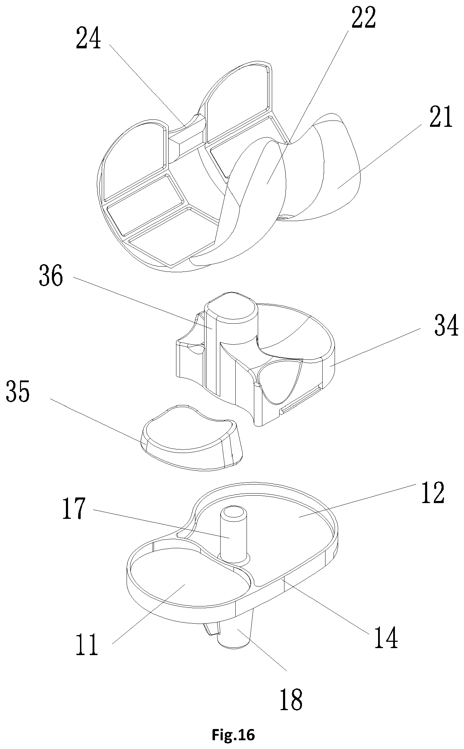

As shown in Fig. 16, in an example that does not form part of the present invention, the medial spacer 34 is fixedly arranged on the tibial plateau 10, and the spacer component 30 further includes a stand column 36 arranged on the medial spacer 34. The stand column 36 may replace a cruciate ligament so as to limit back-forth movement of the femoral condyle, and the stand column 36 has desirable structural strength.

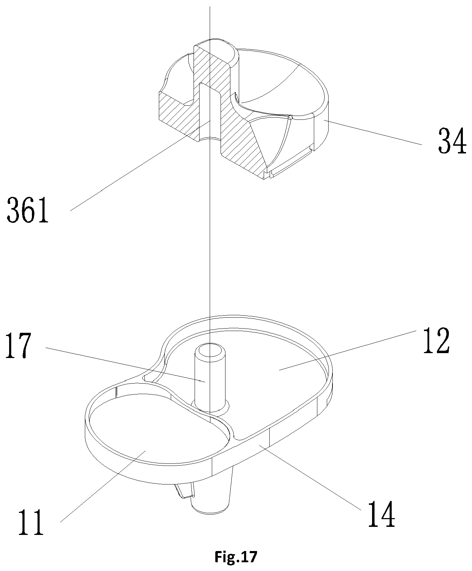

As shown in Figs. 16 and 17, in the example, the femoral condyle component 20 further includes a patellar slideway articular surface 23 connected between the medial condyle articular surface 21 and the lateral condyle articular surface 22, as well as a limiting beam 24 between a posterior condyle of the medial condyle articular surface 21 and a posterior condyle of the lateral condyle articular surface 22. The stand column 36 is capable of limiting the limiting beam 24. The stand column 36 cooperates with the limiting beam 24. The stand column 36 is capable of moving in the limiting beam 24, so as to effectively prevent the knee prosthesis from moving forward excessively.

As shown in Figs. 16 and 17, in the example, the tibial plateau 10 is provided with a shaft 17, the stand column 36 is internally provided with an inner hole 361, and the shaft 17 is inserted into the inner hole 361. The medial spacer 34 is a fixed spacer, and a lower surface of the fixed spacer is flat. The inner hole 361 cooperates with the shaft 17 in a nested manner, so as to enhance mechanical strength of the stand column 36. The stand column 36 is capable of slightly rotating around the shaft 17, and then the spacer component 30 is capable of slightly rotating around the shaft 17, such that the knee prosthesis has more moving positions. In addition, the tibial plateau 10 may be prevented from biting the spacer component 30, and the limiting beam 24 may be limited.

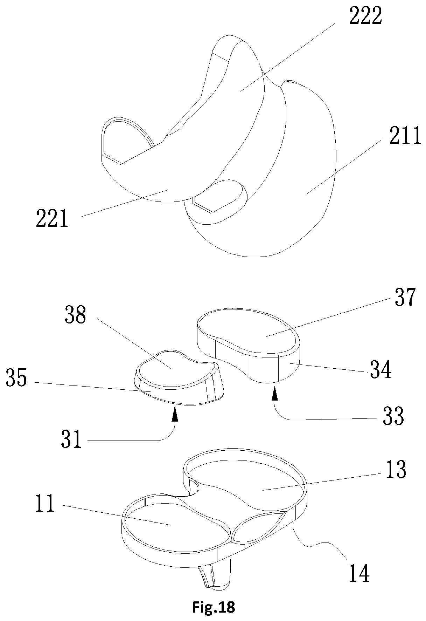

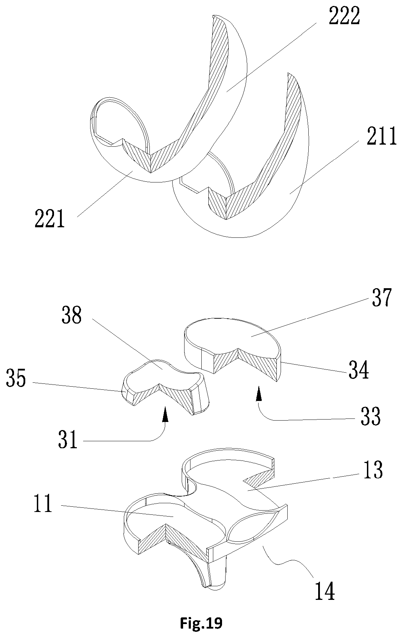

As shown in Figs. 18 and 19, in a second embodiment of the invention, the upper surface of the tibial plateau 10 is provided with a third spherical convex surface 13 at a position corresponding to the medial condyle articular surface 21. A lower surface of the spacer component 30 is provided with a third spherical concave surface 33 cooperating with the third spherical convex surface 13. The third spherical convex surface 13 fits the third spherical convex surface 13. Furthermore, the embodiment provides four spherical friction pairs. Therefore, the design may make the knee prosthesis obtain greater joint mobility and make the whole process from the upright position to the flexed position reliable and stable. Furthermore, a sliding or moving effect of the femoral condyle component 20 is better, thus effectively improving performance of the knee prosthesis.

According to a further example of a knee prosthesis that does not form part of the present invention, the knee prosthesis includes: a tibial plateau 10, a femoral condyle component 20 and a spacer component 30. The femoral condyle component 20 is located above the tibial plateau 10. The femoral condyle component 20 includes a medial condyle articular surface 21 and a lateral condyle articular surface 22. The spacer component 30 is located between the tibial plateau 10 and the femoral condyle component 20. The spacer component 30 includes a medial spacer 34 cooperating with the medial condyle articular surface 21 and a lateral spacer 35 cooperating with the lateral condyle articular surface 22. An upper surface of the tibial plateau 10 is provided with a first spherical convex surface 11 at a position corresponding to the lateral spacer 35. The lateral spacer 35 is movably arranged on the tibial plateau 10. A lower surface of the lateral spacer 35 is provided with a first spherical concave surface 31 cooperating with the first spherical convex surface 11. The example has a difference from the above embodiments in that there is no height difference between the medial condyle articular surface and the lateral condyle articular surface.

In the description of the present invention, it should be understood that orientations or positional relations indicated by the terms "front, rear, upper, lower, left and right", "transverse, vertical, perpendicular, and horizontal", "top and bottom", etc. are based on the orientations or positional relations shown in the accompanying drawings and are only for facilitating the description of the present invention and simplifying the description, and in the absence of a statement to the contrary, the orientations do not indicate or imply that a device or element referred to must have a particular orientation or be constructed and operated in a particular orientation, and therefore will not be interpreted as limiting the scope of protection of the present invention; and the words "inside and outside" refer to inside and outside relative to an outline of each component itself.

For ease of description, spatial relative terms such as "over", "above", "on an upper surface" and "on" may be used herein to describe spatial positional relations of one device or feature with other devices or features as shown in the drawings. It should be understood that the spatial relative terms are intended to include different orientations in use or operation in addition to the orientation of the device described in the drawings. For example, if the device in the drawings is inverted, the device described as "above" or "over" other devices or structures would then be positioned "below" or "under" the other devices or structures. Thus the exemplary term "above" may include two orientations of "above" and "below." The device may also be positioned (rotated 90° or at other orientations) in other different ways and the spatial relative description used herein is interpreted accordingly. In addition, it should be noted that words such as "first" and "second" are used to define parts only for convenience of distinguishing corresponding parts. Unless otherwise stated, the above words have no special meaning, so they cannot be understood as limiting the scope of protection of the present invention.

The foregoing is merely the preferred embodiments of the present invention and not intended to limit the present invention, and various changes and modifications may be made by those skilled in the art. Any modifications or improvements within the ambit of the claims are intended to be included within the scope of protection of the present invention.

## Claims

1. A knee prosthesis, comprising:
a tibial plateau (10);
a femoral condyle component (20), provided above the tibial plateau (10), wherein the femoral condyle component (20) comprises a medial condyle articular surface (21) and a lateral condyle articular surface (22); and
a spacer component (30) between the tibial plateau (10) and the femoral condyle component (20), wherein the spacer component (30) comprises a medial spacer (34) cooperating with the medial condyle articular surface (21) and a lateral spacer (35) cooperating with the lateral condyle articular surface (22), an upper surface of the tibial plateau (10) is provided with a first spherical convex surface (11) at a position corresponding to the lateral spacer (35), the lateral spacer (35) is movably arranged on the tibial plateau (10), and a lower surface of the lateral spacer (35) is provided with a first spherical concave surface (31) cooperating with the first spherical convex surface (11);
**characterized in that**,
the tibial plateau (10) comprises a plateau body (14), a peripheral side wall (15) arranged on a circumferential edge of the plateau body (14), as well as a first medial wall (161) and a second medial wall (162) that are arranged inside the peripheral side wall (15), the lateral spacer (35) is arranged inside a first space surrounded by the first medial wall (161) and the peripheral side wall (15), the medial spacer (34) is arranged inside a second space surrounded by the second medial wall (162) and the peripheral side wall (15), wherein there is a height difference (H) between the medial condyle articular surface (21) and the lateral condyle articular surface (22).

2. The knee prosthesis as claimed in claim 1, wherein the height difference (H) is between 0.5 mm and 5 mm.

3. The knee prosthesis as claimed in claim 1, wherein the lateral condyle articular surface (22) is provided with a second spherical convex surface (221), and the spacer component (30) is provided with a second spherical concave surface (38) cooperating with the second spherical convex surface (221).

4. The knee prosthesis as claimed in claim 1, wherein the lateral condyle articular surface (22) is further provided with a first diameter-variable convex curved surface (222).

5. The knee prosthesis as claimed in claim 1, wherein the medial condyle articular surface (21) is provided with a second diameter-variable convex curved surface (211), and the spacer component (30) is provided with a first diameter-variable concave curved surface (37) cooperating with the second diameter-variable convex curved surface (211).

6. The knee prosthesis as claimed in claim 1, wherein the upper surface of the tibial plateau (10) is provided with a first flat surface (12) at a position corresponding to the medial condyle articular surface (21), and a lower surface of the medial spacer (34) is provided with a second flat surface (32) cooperating with the first flat surface (12); or the upper surface of the tibial plateau (10) is provided with a third spherical convex surface (13) at a position corresponding to the medial condyle articular surface (21), and a lower surface of the medial spacer (34) is provided with a third spherical concave surface (33) cooperating with the third spherical convex surface (13).

7. The knee prosthesis as claimed in claim 1, wherein the first medial wall (161) protrudes toward the lateral spacer (35), and the second medial wall (162) protrudes toward the medial spacer (34).

8. The knee prosthesis as claimed in claim 1, wherein the lateral spacer (35) is movably arranged inside the first space, and the medial spacer (34) is movably arranged inside the second space.

9. The knee prosthesis as claimed in claim 8, wherein a size of the first space is larger than a size of the lateral spacer (35), so as to make the lateral spacer (35) slide and/or rotate relative to the tibial plateau (10), and a size of the second space is larger than a size of the medial spacer (34), so as to make the medial spacer (34) slide and/or rotate relative to the tibial plateau (10).

10. The knee prosthesis as claimed in claim 1, wherein the medial spacer (34) is fixedly arranged on the tibial plateau (10), and the spacer component (30) further comprises a stand column (36) arranged on the medial spacer (34).

11. The knee prosthesis as claimed in claim 10, wherein the femoral condyle component (20) further comprises a patellar slideway articular surface (23) connected between the medial condyle articular surface (21) and the lateral condyle articular surface (22), as well as a limiting beam (24) between a posterior condyle of the medial condyle articular surface (21) and a posterior condyle of the lateral condyle articular surface (22), and the stand column (36) is capable of limiting the limiting beam (24).

12. The knee prosthesis as claimed in claim 11, wherein the tibial plateau (10) is provided with a shaft (17), the stand column (36) is internally provided with an inner hole (361), and the shaft (17) is inserted into the inner hole (361).

13. The knee prosthesis as claimed in claim 7, wherein the tibial plateau (10) further comprises an intramedullary rod (18) arranged below the plateau body (14), and there is an included angle α between a vertical line perpendicular to the upper surface of the tibial plateau (10) and the intramedullary rod (18).

## Patentansprüche

1. Knieprothese, umfassend:
ein Tibiaplateau (10);
eine Femurkondylenkomponente (20), die über dem Tibiaplateau (10) bereitgestellt ist, wobei die Femurkondylenkomponente (20) eine mediale Kondylengelenkfläche (21) und eine laterale Kondylengelenkfläche (22) umfasst; und
eine Abstandshalterkomponente (30) zwischen dem Tibiaplateau (10) und der Femurkondylenkomponente (20), wobei die Abstandshalterkomponente (30) einen medialen Abstandhalter (34), der mit der medialen Kondylengelenkfläche (21) zusammenwirkt, und einen lateralen Abstandhalter (35), der mit der lateralen Kondylengelenkfläche (22) zusammenwirkt, umfasst, wobei eine obere Fläche des Tibiaplateaus (10) mit einer ersten sphärischen konvexen Fläche (11) an einer Position versehen ist, die dem lateralen Abstandhalter (35) entspricht, der laterale Abstandhalter (35) beweglich auf dem Tibiaplateau (10) angeordnet ist und eine untere Fläche des lateralen Abstandhalters (35) mit einer ersten sphärischen konkaven Fläche (31) versehen ist, die mit der ersten sphärischen konvexen Fläche (11) zusammenwirkt;
**dadurch gekennzeichnet, dass**
das Tibiaplateau (10) einen Plateaukörper (14), eine Umfangsseitenwand (15), die an einem umlaufenden Rand des Plateaukörpers (14) angeordnet ist, sowie eine erste mediale Wand (161) und eine zweite mediale Wand (162) umfasst, die innerhalb der Umfangsseitenwand (15) angeordnet sind, der laterale Abstandshalter (35) innerhalb eines ersten Raums angeordnet ist, der von der ersten medialen Wand (161) und der Umfangsseitenwand (15) umgeben ist, der mediale Abstandshalter (34) innerhalb eines zweiten Raums angeordnet ist, der von der zweiten medialen Wand (162) und der Umfangsseitenwand (15) umgeben ist, wobei eine Höhendifferenz (H) zwischen der medialen Kondylengelenkfläche (21) und der lateralen Kondylengelenkfläche (22) besteht.

2. Knieprothese nach Anspruch 1, wobei die Höhendifferenz (H) zwischen 0,5 mm und 5 mm beträgt.

3. Knieprothese nach Anspruch 1, wobei die laterale Kondylengelenkfläche (22) mit einer zweiten sphärischen konvexen Fläche (221) versehen ist und die Abstandshalterkomponente (30) mit einer zweiten sphärischen konkaven Fläche (38) versehen ist, die mit der zweiten sphärischen konvexen Fläche (221) zusammenwirkt.

4. Knieprothese nach Anspruch 1, wobei die laterale Kondylengelenkfläche (22) ferner mit einer ersten durchmesserveränderlichen konvex gekrümmten Fläche (222) versehen ist.

5. Knieprothese nach Anspruch 1, wobei die mediale Kondylengelenkfläche (21) mit einer zweiten durchmesserveränderlichen konvexen gekrümmten Fläche (211) versehen ist und die Abstandshalterkomponente (30) mit einer ersten durchmesserveränderlichen konkaven gekrümmten Fläche (37) versehen ist, die mit der zweiten durchmesserveränderlichen konvexen gekrümmten Fläche (211) zusammenwirkt.

6. Knieprothese nach Anspruch 1, wobei die obere Fläche des Tibiaplateaus (10) mit einer ersten flachen Fläche (12) an einer Position versehen ist, die der medialen Kondylengelenkfläche (21) entspricht, und eine untere Fläche des medialen Abstandshalters (34) mit einer zweiten flachen Fläche (32) versehen ist, die mit der ersten flachen Fläche (12) zusammenwirkt; oder die obere Fläche des Tibiaplateaus (10) ist mit einer dritten sphärischen konvexen Fläche (13) an einer Position versehen, die der medialen Kondylengelenkfläche (21) entspricht, und eine untere Fläche des medialen Abstandshalters (34) ist mit einer dritten sphärischen konkaven Fläche (33) versehen, die mit der dritten sphärischen konvexen Fläche (13) zusammenwirkt.

7. Knieprothese nach Anspruch 1, wobei die erste mediale Wand (161) in Richtung des lateralen Abstandshalters (35) vorsteht und die zweite mediale Wand (162) in Richtung des medialen Abstandshalters (34) vorsteht.

8. Knieprothese nach Anspruch 1, wobei der laterale Abstandshalter (35) beweglich innerhalb des ersten Raums angeordnet ist und der mediale Abstandshalter (34) beweglich innerhalb des zweiten Raums angeordnet ist.

9. Knieprothese nach Anspruch 8, wobei eine Größe des ersten Raums größer als eine Größe des lateralen Abstandshalters (35) ist, sodass der laterale Abstandshalter (35) relativ zum Tibiaplateau (10) gleiten und/oder sich drehen kann, und eine Größe des zweiten Raums größer als eine Größe des medialen Abstandshalters (34) ist, sodass der mediale Abstandshalter (34) relativ zum Tibiaplateau (10) gleiten und/oder sich drehen kann.

10. Knieprothese nach Anspruch 1, wobei der mediale Abstandshalter (34) fest auf dem Tibiaplateau (10) angeordnet ist und die Abstandshalterkomponente (30) ferner eine auf dem medialen Abstandshalter (34) angeordnete Standsäule (36) umfasst.

11. Knieprothese nach Anspruch 10, wobei die Femurkondylenkomponente (20) ferner eine Patellagleitbahn-Gelenkfläche (23), die zwischen der medialen Kondylengelenkfläche (21) und der lateralen Kondylengelenkfläche (22) verbunden ist, sowie einen Begrenzungsbalken (24) zwischen einer posterioren Kondyl der medialen Kondylengelenkfläche (21) und einer posterioren Kondyl der lateralen Kondylengelenkfläche (22) umfasst und die Standsäule (36) in der Lage ist, den Begrenzungsbalken (24) zu begrenzen.

12. Knieprothese nach Anspruch 11, wobei das Tibiaplateau (10) mit einem Schaft (17) versehen ist, die Standsäule (36) innen mit einem Innenloch (361) versehen ist und der Schaft (17) in das Innenloch (361) eingesetzt ist.

13. Knieprothese nach Anspruch 7, wobei das Tibiaplateau (10) ferner eine intramedulläre Stange (18) umfasst, die unterhalb des Plateaukörpers (14) angeordnet ist, und ein eingeschlossener Winkel α zwischen einer vertikalen Linie senkrecht zur oberen Fläche des Tibiaplateaus (10) und der intramedullären Stange (18) besteht.

## Revendications

1. Prothèse de genou, comprenant :
un plateau tibial (10) ;
un composant de condyle fémoral (20), placé au-dessus du plateau tibial (10), dans laquelle le composant du condyle fémoral (20) comprend une surface articulaire de condyle médial (21) et une surface articulaire de condyle latéral (22) ; et
un composant d'entretoise (30) entre le plateau tibial (10) et le composant du condyle fémoral (20), dans laquelle le composant d'entretoise (30) comprend une entretoise médiale (34) coopérant avec la surface articulaire du condyle médial (21) et une entretoise latérale (35) coopérant avec la surface articulaire du condyle latéral (22), une surface supérieure du plateau tibial (10) est pourvue d'une première surface convexe sphérique (11) à une position correspondant à l'entretoise latérale (35), l'entretoise latérale (35) est disposée de manière mobile sur le plateau tibial (10), et une surface inférieure de l'entretoise latérale (35) est pourvue d'une première surface concave sphérique (31) coopérant avec la première surface convexe sphérique (11) ;
**caractérisé en ce que**
le plateau tibial (10) comprend un corps de plateau (14), une paroi latérale périphérique (15) disposée sur un bord circonférentiel du corps de plateau (14), ainsi qu'une première paroi médiale (161) et une seconde paroi médiale (162) qui sont disposées à l'intérieur de la paroi latérale périphérique (15), l'entretoise latérale (35) est disposée à l'intérieur d'un premier espace entouré par la première paroi médiale (161) et la paroi latérale périphérique (15), l'entretoise médiale (34) est disposée à l'intérieur d'un second espace entouré par la seconde paroi médiale (162) et la paroi latérale périphérique (15), dans laquelle il existe une différence de hauteur (H) entre la surface articulaire du condyle médial (21) et la surface articulaire du condyle latéral (22).

2. Prothèse de genou selon la revendication 1, dans laquelle la différence de hauteur (H) est comprise entre 0,5 et 5 mm.

3. Prothèse de genou selon la revendication 1, dans laquelle la surface articulaire du condyle latéral (22) est pourvue d'une deuxième surface convexe sphérique (221), et le composant d'entretoise (30) est pourvu d'une deuxième surface concave sphérique (38) coopérant avec la deuxième surface convexe sphérique (221).

4. Prothèse de genou selon la revendication 1, dans laquelle la surface articulaire du condyle latéral (22) est en outre pourvue d'une première surface convexe incurvée à diamètre variable (222).

5. Prothèse de genou selon la revendication 1, dans laquelle la surface articulaire du condyle médial (21) est pourvue d'une seconde surface incurvée convexe à diamètre variable (211), et le composant d'entretoise (30) est pourvu d'une première surface incurvée concave à diamètre variable (37) coopérant avec la seconde surface incurvée convexe à diamètre variable (211).

6. Prothèse de genou selon la revendication 1, dans laquelle la surface supérieure du plateau tibial (10) est pourvue d'une première surface plate (12) à une position correspondant à la surface articulaire du condyle médial (21), et une surface inférieure de l'entretoise médiale (34) est pourvue d'une seconde surface plate (32) coopérant avec la première surface plate (12) ; ou la surface supérieure du plateau tibial (10) est pourvue d'une troisième surface convexe sphérique (13) à une position correspondant à la surface articulaire du condyle médial (21), et une surface inférieure de l'entretoise médiale (34) est pourvue d'une troisième surface concave sphérique (33) coopérant avec la troisième surface convexe sphérique (13).

7. Prothèse de genou selon la revendication 1, dans laquelle la première paroi médiale (161) fait saillie vers l'entretoise latérale (35), et la seconde paroi médiale (162) fait saillie vers l'entretoise médiale (34).

8. Prothèse de genou selon la revendication 1, dans laquelle l'entretoise latérale (35) est disposée de manière mobile à l'intérieur du premier espace, et l'entretoise médiale (34) est disposée de manière mobile à l'intérieur du second espace.

9. Prothèse de genou selon la revendication 8, dans laquelle une taille du premier espace est supérieure à une taille de l'entretoise latérale (35), de manière à faire glisser et/ou tourner l'entretoise latérale (35) par rapport au plateau tibial (10), et une taille du second espace est supérieure à une taille de l'entretoise médiale (34), de manière à faire glisser et/ou tourner l'entretoise médiale (34) par rapport au plateau tibial (10).

10. Prothèse de genou selon la revendication 1, dans laquelle l'entretoise médiale (34) est disposée de manière fixe sur le plateau tibial (10), et le composant d'entretoise (30) comprend en outre une colonne de support (36) disposée sur l'entretoise médiale (34).

11. Prothèse de genou selon la revendication 10, dans laquelle le composant du condyle fémoral (20) comprend en outre une surface articulaire de glissement rotulien (23) reliée entre la surface articulaire du condyle médial (21) et la surface articulaire du condyle latéral (22), ainsi qu'une barre de limitation (24) entre un condyle postérieur de la surface articulaire du condyle médial (21) et un condyle postérieur de la surface articulaire du condyle latéral (22), et la colonne de support (36) est capable de limiter la barre de limitation (24).

12. Prothèse de genou selon la revendication 11, dans laquelle le plateau tibial (10) est pourvu d'une tige (17), la colonne de support (36) est pourvue intérieurement d'un trou intérieur (361), et la tige (17) est insérée dans le trou intérieur (361).

13. Prothèse de genou selon la revendication 7, dans laquelle le plateau tibial (10) comprend en outre une tige intramédullaire (18) disposée sous le corps de plateau (14), et il existe un angle inclus α entre une ligne verticale perpendiculaire à la surface supérieure du plateau tibial (10) et la tige intramédullaire (18).
